# EUROPEAN PATENT APPLICATION

(11) **EP 4 850 659 A1**
(43) Date of publication of application: **09.12.2026**
(21) Application number: 24922078.1
(22) Date of filing: 25.09.2024
(51) Int. Cl.: A61K 8/19, A61K 8/20, A61K 8/26, A61K 8/34, A61K 8/36, A61K 8/37, A61K 8/39, A61K 8/46, A61K 8/86, A61Q 11/00

(54) **DENTURE STABILIZER REMOVER**

(30) Priority: 30.01.2024 JP 2024011551
(71) Applicant: Nippon Shika Yakuhin Co., Ltd., Yamaguchi 750-0025 (JP)
(72) Inventor: YAMAGUCHI Tomomasa, Shimonoseki-shi, Yamaguchi 750-0025 (JP); TESHIMA Hiroki, Shimonoseki-shi, Yamaguchi 750-0025 (JP)
(74) Representative: Müller-Boré & Partner Patentanwälte PartG mbB
(86) International application number: PCT/JP2024/034285
(87) International publication number: WO 2025/163966

(57) **Abstract**

A denture adhesive remover comprising a polar base and at least one metal compound, wherein the metal compound is a compound selected from the group consisting of:
(1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
(2) an oxide of at least one metal selected from the group consisting of sodium, potassium, calcium, magnesium, aluminum, zinc, and iron, or a hydrate thereof; and
(3) a hydroxide of at least one metal selected from the group consisting of sodium, potassium, calcium, magnesium, aluminum, zinc, and iron, or a hydrate thereof
is able to sufficiently remove a denture adhesive attached to a denture.

## Description

### Technical Field

The present invention relates to a denture adhesive remover for removing a denture adhesive attached to the oral cavity or a denture.

### Background Art

A denture adhesive may be used when a denture is inserted into the oral cavity. The denture adhesive is applied onto the surface of the denture base as the base portion of a denture and increases the tackiness or adhesion to the gum or the upper jaw to prevent the denture from shifting while eating or conversation.

The denture adhesive includes various types such as a cream type, a powder type, and a cushion type. Specifically, "Poligrip (registered trademark)", which is classified into the cream type, holds the top market share in denture adhesives and is widely sold not only in dental clinics but also in general drugstores.

The denture adhesive absorbs saliva in the oral cavity to exert tackiness and improves the retention and stability of a denture. The main ingredient for the tackiness is a water-swellable, water-soluble polymer compound such as "sodium/calcium methoxyethylene-maleic anhydride copolymer salt (PVMA salt)" and "carboxymethyl cellulose (CMC)". Cream-type denture adhesives, such as Poligrip, further comprise a hydrophobic ointment base ingredient such as "liquid paraffin" and "white petrolatum". The denture adhesive is retained in the oral cavity through a balance between the water absorption and viscosity exerted by the water-soluble polymer and the dissolution resistance by the hydrophobic ointment base ingredient.

A denture and a denture adhesive are not permanently retained in the oral cavity but are required to be regularly removed from the oral cavity for cleansing. The current method for cleansing dentures includes mechanical cleansing using brushes and the like and chemical cleansing using cleansers. Each cleansing method is desirable to avoid scratching or deforming dentures.

It is, however, not easy to completely remove the denture adhesive attached to the oral cavity or a denture. This is due to some reasons: for example, a denture adhesive having high tackiness is strongly attached to the oral cavity or a denture; and applying strong force to thoroughly remove the denture adhesive may lead to bleeding from the oral mucosa or to patient pain or may scratch and deform a denture. This makes aggressive cleansing difficult both practically and psychologically. The complicated structures of teeth and tooth necks in the oral cavity and the unevenness of a denture also make it difficult to remove a denture adhesive.

Specifically in nursing care settings, some denture wearers cannot cooperate, or sufficient time and effort are often difficult to devote. Hence, a denture adhesive not completely removed may be found in the oral cavity or on a denture.

On the surface of a denture adhesive remaining in the oral cavity or on a denture, bacteria can proliferate to form a biofilm, which may cause denture stomatitis. In addition, it has been suggested that oral bacteria may be associated with other diseases such as bacterial endocarditis, aspiration pneumonia, and respiratory tract infections. Many denture wearers are elderly, often have poor general physical conditions, and thus are susceptible to these diseases. There is therefore a need for effective removal of denture adhesives.

Some denture adhesive removers are known. For example, Patent Literature 1 discloses a denture adhesive remover comprising 30 to 60% by weight of a lower alcohol, water, a thickener, and a surfactant. The literature discloses that the denture adhesive remover can effectively remove a denture adhesive and has a bactericidal effect.

The remover taught in Patent Literature 1 however cannot completely remove a denture adhesive from a denture even when force is applied to the denture and the denture adhesive coated with the remover.

### Citation List

### Patent Literature

Patent Literature 1: JP 2020-164500 A

### Summary of Invention

### Technical Problem

The present invention has an objective to provide a denture adhesive remover capable of sufficiently removing a denture adhesive attached to a denture.

### Solution to Problem

In order to achieve the objective, the present invention has focused on the chemical properties of a denture adhesive. The inventors have thought that by chemically inhibiting the function of a water-swellable, water-soluble polymer compound contained in a denture adhesive to reduce the tackiness or adhesiveness, the denture adhesive attached to a denture can be effectively removed. The inventors have found that a formulation containing a polar base and a particular metal compound can sufficiently remove the denture adhesive attached to a denture.

The present invention has been completed on the basis of the above findings and provides the following aspects [1] to [14].
[1] A denture adhesive remover comprising a polar base and at least one metal compound, wherein the metal compound is a compound selected from the group consisting of:
   (1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
   (2) an oxide of at least one metal selected from the group consisting of sodium, potassium, calcium, magnesium, aluminum, zinc, and iron, or a hydrate thereof; and
   (3) a hydroxide of at least one metal selected from the group consisting of sodium, potassium, calcium, magnesium, aluminum, zinc, and iron, or a hydrate thereof.
[2] The denture adhesive remover according to the aspect [1], wherein the inorganic acid is a compound selected from the group consisting of hydrochloric acid, carbonic acid, nitric acid, nitrous acid, sulfuric acid, sulfurous acid, thiosulfuric acid, silicic acid, phosphoric acid, and hydrofluoric acid, and the organic acid is a compound selected from the group consisting of lactic acid, acetic acid, citric acid, gluconic acid, and hexacyanoferric acid.
[3] The denture adhesive remover according to the aspect [1] or [2], wherein the polar base is at least one selected from the group consisting of lower alcohols, higher alcohols, polyhydric alcohols, and glycol ethers.
[4] The denture adhesive remover according to any one of the aspects [1] to [3], wherein the concentration of the polar base is 1 to 99% by mass relative to the total amount of the denture adhesive remover.
[5] The denture adhesive remover according to any one of the aspects [1] to [4], wherein the concentration of the metal compound is 0.01 to 97% by mass relative to the total amount of the denture adhesive remover.
[6] The denture adhesive remover according to any one of the aspects [1] to [5], wherein the content of the metal compound is 0.0005 to 20 parts by mass relative to 1 part by mass of the polar base.
[7] The denture adhesive remover according to any one of the aspects [1] to [6], further comprising a surfactant.
[8] The denture adhesive remover according to the aspect [7], wherein the concentration of the surfactant is 0.01 to 20% by mass relative to the total amount of the denture adhesive remover.
[9] The denture adhesive remover according to the aspect [7] or [8], wherein the content of the surfactant is 0.01 to 20 parts by mass relative to 1 part by mass of the metal compound.
[10] The denture adhesive remover according to any one of the aspects [1] to [9], further comprising water.
[11] The denture adhesive remover according to the aspect [10], wherein the concentration of the water is 0.001 to 90% by mass relative to the total amount of the denture adhesive remover.
[12] The denture adhesive remover according to any one of the aspects [1] to [11], for removing a denture adhesive from a denture or the oral cavity.
[13] Use of a composition as a denture adhesive remover or use of a composition for the manufacture of a denture adhesive remover, the composition comprising a polar base and at least one metal compound, wherein the metal compound is a compound selected from the group consisting of:
   (1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
   (2) an oxide of at least one metal selected from the group consisting of sodium, potassium, calcium, magnesium, aluminum, zinc, and iron, or a hydrate thereof; and
   (3) a hydroxide of at least one metal selected from the group consisting of sodium, potassium, calcium, magnesium, aluminum, zinc, and iron, or a hydrate thereof.
[14] A method for removing a denture adhesive, the method comprising a step of applying an effective dose of a composition to a denture adhesive, the composition comprising a polar base and at least one metal compound, wherein the metal compound is a compound selected from the group consisting of:
   (1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
   (2) an oxide of at least one metal selected from the group consisting of sodium, potassium, calcium, magnesium, aluminum, zinc, and iron, or a hydrate thereof; and
   (3) a hydroxide of at least one metal selected from the group consisting of sodium, potassium, calcium, magnesium, aluminum, zinc, and iron, or a hydrate thereof.

### Advantageous Effects of Invention

Although conventional denture adhesive removers (hereinafter also abbreviated as "removers") fail to completely remove a denture adhesive adhering to a denture even when strong force is applied, the denture adhesive remover of the present invention chemically reduces the tackiness of a denture adhesive and thus can remove a denture adhesive by applying less force as compared to conventional removers or without applying force.

The remover of the present invention works to change properties of a denture adhesive and accordingly removes the denture adhesive. Hence, the remover can remove a denture adhesive from not only a denture but also the oral mucosa. The remover can sufficiently remove a denture adhesive even when comprising neither irritating ingredients nor ingredients that may cause unfavorable effects on the human body. Hence, the remover is suited for removing a denture adhesive attached or adhering to the oral mucosa. Conventional removers remove a denture adhesive attached to a denture that has been detached from the oral cavity, and there is no remover that can remove a denture adhesive attached to the oral cavity. The remover of the present invention can effectively remove a denture adhesive attached to the oral cavity and thus has a high commercial value.

In many denture adhesives, a carboxyl group-containing polymer comes into contact with water to ionize, and the negatively charged polymer electrostatically interacts with the denture base and the oral mucosa and strongly adheres to the denture base and the oral mucosa. Without limitation, the mechanism of the remover of the present invention is thought as follows: the polar base suppresses the electrostatic interactions and thus weakens the tackiness of a denture adhesive to a denture.

Without limitation, the following mechanism is also thought: a carboxyl group-containing polymer contained in many denture adhesives comes into contact with water to ionize and is negatively charged, and intramolecular/intermolecular electrostatic repulsion and steric hindrance increase the viscosity of the polymer; but metal ions work to reduce the viscosity of the polymer, resulting in easy detachment from a denture and the oral cavity.

### Brief Description of Drawings

Fig. 1 is photographs in the oral cavity before and after cleansing a denture adhesive attached to the oral cavity by using the formulation in Example 57.
Fig. 2 is photographs in the oral cavity before and after cleansing a denture adhesive attached to the oral cavity by using the formulation in Comparative Example 2.

### Description of Embodiments

The present invention will now be described in detail.

A denture adhesive remover of the present invention comprises a polar base and at least one metal compound, and the metal compound is a compound selected from the group consisting of:
(1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
(2) an oxide of at least one metal selected from the group consisting of sodium, potassium, calcium, magnesium, aluminum, zinc, and iron, or a hydrate thereof; and
(3) a hydroxide of at least one metal selected from the group consisting of sodium, potassium, calcium, magnesium, aluminum, zinc, and iron, or a hydrate thereof.

### Polar base

As the polar base, a polar base used in an external composition may be used, and examples include lower alcohols (alcohols having a carbon number of 1 to 5, preferably a carbon number of 2 to 4), higher alcohols (aliphatic alcohols having a carbon number of 6 to 24, preferably a carbon number of 12 to 18), polyhydric alcohols, and glycol ethers.

One or more polar bases may be used.

Polar bases are in various states such as liquid, paste, powder, and flakes at normal temperature depending on the type or the molecular weight, and combination use enables control of the remover state.

Examples of the lower alcohol include methanol, ethanol, propyl alcohol, isopropyl alcohol, butanol, and amyl alcohol.

Examples of the higher alcohol include linear saturated higher alcohols such as hexyl alcohol, heptyl alcohol, capryl alcohol, nonyl alcohol, decyl alcohol, undecanol, lauryl alcohol, myristyl alcohol, cetanol, cetearyl alcohol, stearyl alcohol, arachidyl alcohol, and behenyl alcohol; linear unsaturated higher alcohols such as oleyl alcohol, linoleyl alcohol, and lignoceryl alcohol; and branched-chain saturated higher alcohols such as hexyldecanol, isostearyl alcohol, octyldodecanol, and decyltetradecanol.

Examples of the polyhydric alcohol include propylene glycol, dipropylene glycol, polypropylene glycol, 1,3-butylene glycol, glycerol, diglycerol, isoprene glycol, and polyethylene glycol. The polyhydric alcohol may be a polymer such as polypropylene glycol and polyethylene glycol or may be a non-polymer, but a polymer is preferred.

Examples of the glycol ether include ethylene glycol monomethyl ether, ethylene glycol monoethyl ether, ethylene glycol monopropyl ether, diethylene glycol monomethyl ether, diethylene glycol monoethyl ether, diethylene glycol monopropyl ether, diethylene glycol monobutyl ether, propylene glycol monoethyl ether, propylene glycol monopropyl ether, dipropylene glycol monoethyl ether, and dipropylene glycol monopropyl ether.

The polar base is preferably a lower alcohol or a polyhydric alcohol and is specifically preferably ethanol, isopropyl alcohol, polypropylene glycol, or polyethylene glycol.

The concentration of the polar base relative to the total amount of the remover may be 1% by mass or more, 5% by mass or more, 10% by mass or more, 20% by mass or more, 30% by mass or more, 40% by mass or more, or 50% by mass or more. Within the range, denture adhesives are effectively removed.

The concentration of the polar base relative to the total amount of the remover may be 99% by mass or less, 98% by mass or less, 97% by mass or less, 96% by mass or less, 95% by mass or less, 93% by mass or less, 90% by mass or less, 80% by mass or less, 70% by mass or less, or 60% by mass or less. Within the range, a sufficient amount of the metal compound or other ingredients may be further contained, and the resulting formulation exhibits high efficacy in removing denture adhesives.

Examples of the concentration of the polar base relative to the total amount of the remover include 1 to 99% by mass, 1 to 98% by mass, 1 to 97% by mass, 1 to 96% by mass, 1 to 95% by mass, 1 to 93% by mass, 1 to 90% by mass, 1 to 80% by mass, 1 to 70% by mass, 1 to 60% by mass; 5 to 99% by mass, 5 to 98% by mass, 5 to 97% by mass, 5 to 96% by mass, 5 to 95% by mass, 5 to 93% by mass, 5 to 90% by mass, 5 to 80% by mass, 5 to 70% by mass, 5 to 60% by mass; 10 to 99% by mass, 10 to 98% by mass, 10 to 97% by mass, 10 to 96% by mass, 10 to 95% by mass, 10 to 93% by mass, 10 to 90% by mass, 10 to 80% by mass, 10 to 70% by mass, 10 to 60% by mass; 20 to 99% by mass, 20 to 98% by mass, 20 to 97% by mass, 20 to 96% by mass, 20 to 95% by mass, 20 to 93% by mass, 20 to 90% by mass, 20 to 80% by mass, 20 to 70% by mass, 20 to 60% by mass; 30 to 99% by mass, 30 to 98% by mass, 30 to 97% by mass, 30 to 96% by mass, 30 to 95% by mass, 30 to 93% by mass, 30 to 90% by mass, 30 to 80% by mass, 30 to 70% by mass, 30 to 60% by mass; 40 to 99% by mass, 40 to 98% by mass, 40 to 97% by mass, 40 to 96% by mass, 40 to 95% by mass, 40 to 93% by mass, 40 to 90% by mass, 40 to 80% by mass, 40 to 70% by mass, 40 to 60% by mass; 50 to 99% by mass, 50 to 98% by mass, 50 to 97% by mass, 50 to 96% by mass, 50 to 95% by mass, 50 to 93% by mass, 50 to 90% by mass, 50 to 80% by mass, 50 to 70% by mass, and 50 to 60% by mass.

For a remover comprising a polyhydric alcohol, the concentration of the polyhydric alcohol may be 1% by mass or more, 5% by mass or more, 10% by mass or more, 20% by mass or more, 30% by mass or more, 40% by mass or more, or 50% by mass or more. Within the range, denture adhesives are effectively removed.

The concentration of the polyhydric alcohol relative to the total amount of the remover may be 99% by mass or less, 98% by mass or less, 97% by mass or less, 96% by mass or less, 95% by mass or less, 93% by mass or less, 90% by mass or less, 80% by mass or less, 70% by mass or less, or 60% by mass or less. Within the range, a sufficient amount of the metal compound or other ingredients may be further contained, and the resulting formulation exhibits high efficacy in removing denture adhesives.

Examples of the concentration of the polyhydric alcohol relative to the total amount of the remover include 1 to 99% by mass, 1 to 98% by mass, 1 to 97% by mass, 1 to 96% by mass, 1 to 95% by mass, 1 to 93% by mass, 1 to 90% by mass, 1 to 80% by mass, 1 to 70% by mass, 1 to 60% by mass; 5 to 99% by mass, 5 to 98% by mass, 5 to 97% by mass, 5 to 96% by mass, 5 to 95% by mass, 5 to 93% by mass, 5 to 90% by mass, 5 to 80% by mass, 5 to 70% by mass, 5 to 60% by mass; 10 to 99% by mass, 10 to 98% by mass, 10 to 97% by mass, 10 to 96% by mass, 10 to 95% by mass, 10 to 93% by mass, 10 to 90% by mass, 10 to 80% by mass, 10 to 70% by mass, 10 to 60% by mass; 20 to 99% by mass, 20 to 98% by mass, 20 to 97% by mass, 20 to 96% by mass, 20 to 95% by mass, 20 to 93% by mass, 20 to 90% by mass, 20 to 80% by mass, 20 to 70% by mass, 20 to 60% by mass; 30 to 99% by mass, 30 to 98% by mass, 30 to 97% by mass, 30 to 96% by mass, 30 to 95% by mass, 30 to 93% by mass, 30 to 90% by mass, 30 to 80% by mass, 30 to 70% by mass, 30 to 60% by mass; 40 to 99% by mass, 40 to 98% by mass, 40 to 97% by mass, 40 to 96% by mass, 40 to 95% by mass, 40 to 93% by mass, 40 to 90% by mass, 40 to 80% by mass, 40 to 70% by mass, 40 to 60% by mass; 50 to 99% by mass, 50 to 98% by mass, 50 to 97% by mass, 50 to 96% by mass, 50 to 95% by mass, 50 to 93% by mass, 50 to 90% by mass, 50 to 80% by mass, 50 to 70% by mass, and 50 to 60% by mass.

For a remover comprising a lower alcohol, the concentration of the lower alcohol may be 5% by mass or more, 10% by mass or more, 20% by mass or more, 25% by mass or more, 30% by mass or more, 40% by mass or more, or 50% by mass or more. Within the range, denture adhesives are effectively removed.

The concentration of the lower alcohol relative to the total amount of the remover may be 40% by mass or less, 35% by mass or less, 30% by mass or less, 20% by mass or less, 15% by mass or less, 10% by mass or less, or 5% by mass or less. Within the range, a sufficient amount of the metal compound may be further contained, and the resulting formulation exhibits high efficacy in removing denture adhesives. When the concentration is specifically 10% by mass or less, irritation to the oral mucosa is suppressed, and the resulting denture adhesive remover becomes more suitable for use in the oral cavity.

Examples of the concentration of the lower alcohol relative to the total amount of the remover include 5 to 40% by mass, 5 to 35% by mass, 5 to 30% by mass, 5 to 20% by mass, 5 to 15% by mass, 5 to 10% by mass; 10 to 40% by mass, 10 to 35% by mass, 10 to 30% by mass, 10 to 20% by mass, 10 to 15% by mass; 20 to 40% by mass, 20 to 35% by mass, 20 to 30% by mass; 25 to 40% by mass, 25 to 35% by mass, 25 to 30% by mass; 30 to 40% by mass, and 30 to 35% by mass.

### Metal compound

Examples of the metal compound include salts of at least one metal selected from sodium, potassium, calcium, magnesium, aluminum, zinc, and iron with at least one selected from inorganic acids, organic acids, and ammonia and hydrates thereof. Examples of the inorganic acid include hydrochloric acid, carbonic acid, phosphoric acid, nitric acid, nitrous acid, sulfuric acid, sulfurous acid, thiosulfuric acid, silicic acid, and hydrofluoric acid. From the inorganic acid salts, phosphates may be excluded, and specifically calcium phosphate may be excluded. Examples of the organic acid include lactic acid, acetic acid, citric acid, hexacyanoferric acid, and gluconic acid. From the organic acid salts, stearates may be excluded, and specifically calcium stearate may be excluded.

Examples of the metal compound also include oxides of at least one metal selected from sodium, potassium, calcium, magnesium, aluminum, zinc, and iron and hydrates thereof. From the oxides, magnesium oxide may be excluded.

Examples of the metal compound also include hydroxides of at least one metal selected from sodium, potassium, calcium, magnesium, aluminum, zinc, and iron and hydrates thereof.

In the metal compound, two or more metals may be contained, and two or more counter ions may be contained for a metal ion. Examples include salts of the metal with an organic acid or an inorganic acid and with ammonia.

Examples of the metal compound include sodium chloride, sodium carbonate, sodium hydrogen carbonate, sodium sulfate (including anhydrous sodium sulfate), sodium sulfite, sodium thiosulfate, sodium acetate, sodium nitrite, sodium fluoride, sodium lactate, sodium acetate, sodium citrate, sodium gluconate, sodium hydroxide; potassium chloride, potassium fluoride, potassium carbonate, potassium hydrogen carbonate, potassium phosphate, potassium lactate, potassium acetate, potassium citrate, potassium gluconate, potassium hydroxide, aluminum potassium sulfate; calcium sulfate, calcium nitrate, calcium hydrogen carbonate, calcium chloride, calcium oxide, calcium lactate, calcium citrate, calcium acetate ; magnesium chloride, magnesium sulfate, magnesium nitrate, magnesium citrate, magnesium acetate; aluminum chloride, aluminum sulfate, aluminum phosphate, aluminum ammonium sulfate, aluminum nitrate, aluminum oxide, aluminum lactate; zinc chloride, zinc fluoride, zinc sulfate, zinc nitrate, zinc gluconate; iron chloride, iron sulfate, ammonium iron sulfate, potassium hexacyanoferrate (including potassium hexacyanoferrate(III)); and hydrates thereof.

Of them, preferred examples of the metal compound include sodium carbonate, sodium chloride, sodium sulfate (including anhydrous sodium sulfate), sodium sulfite, sodium thiosulfate, sodium hydroxide; potassium fluoride, potassium hexacyanoferrate(III), potassium hydroxide, aluminum potassium sulfate, potassium acetate; calcium acetate, calcium chloride, calcium lactate, calcium oxide; magnesium chloride, magnesium nitrate, magnesium sulfate, magnesium acetate; aluminum lactate, aluminum sulfate, aluminum phosphate, aluminum ammonium sulfate, aluminum oxide; zinc fluoride; zinc chloride, zinc fluoride, zinc nitrate; iron chloride, iron sulfate, ammonium iron sulfate, potassium hexacyanoferrate (including potassium hexacyanoferrate(III)); and hydrates thereof. These metal compounds provide particularly excellent removal effect.

For example, when a compound causing no or little irritation to the oral mucosa or no or little unpleasant taste is selected from these compounds, the resulting formulation becomes more suitable as the remover of denture adhesives from the oral cavity.

One or more metal compounds may be used. The metal compound used in the present invention may be a compound that generates metal ions (particularly, a certain amount of metal ions) in the remover or in the remover mixed with water for use.

In the remover, the concentration of the metal compound relative to the total amount of the remover may be 0.01% by mass or more, 0.05% by mass or more, 0.1% by mass or more, 0.5% by mass or more, 1% by mass or more, 5% by mass or more, 10% by mass or more, or 20% by mass or more. Within the range, denture adhesives are effectively removed.

In the remover, the concentration of the metal compound relative to the total amount of the remover may be 97% by mass or less, 95% by mass or less, 90% by mass or less, 80% by mass or less, 70% by mass or less, 60% by mass or less, 50% by mass or less, 40% by mass or less, or 30% by mass or less. Within the range, a sufficient amount of the polar base or other ingredients may be further contained, and the resulting formulation exhibits high efficacy in removing denture adhesives.

Examples of the concentration of the metal compound relative to the total amount of the remover include 0.01 to 97% by mass, 0.01 to 95% by mass, 0.01 to 90% by mass, 0.01 to 80% by mass, 0.01 to 70% by mass, 0.01 to 60% by mass, 0.01 to 50% by mass, 0.01 to 40% by mass, 0.01 to 30% by mass; 0.05 to 97% by mass, 0.05 to 95% by mass, 0.05 to 90% by mass, 0.05 to 80% by mass, 0.05 to 70% by mass, 0.05 to 60% by mass, 0.05 to 50% by mass, 0.05 to 40% by mass, 0.05 to 30% by mass; 0.1 to 97% by mass, 0.1 to 95% by mass, 0.1 to 90% by mass, 0.1 to 80% by mass, 0.1 to 70% by mass, 0.1 to 60% by mass, 0.1 to 50% by mass, 0.1 to 40% by mass, 0.1 to 30% by mass; 0.5 to 97% by mass, 0.5 to 95% by mass, 0.5 to 90% by mass, 0.5 to 80% by mass, 0.5 to 70% by mass, 0.5 to 60% by mass, 0.5 to 50% by mass, 0.5 to 40% by mass, 0.5 to 30% by mass; 1 to 97% by mass, 1 to 95% by mass, 1 to 90% by mass, 1 to 80% by mass, 1 to 70% by mass, 1 to 60% by mass, 1 to 50% by mass, 1 to 40% by mass, 1 to 30% by mass; 5 to 97% by mass, 5 to 95% by mass, 5 to 90% by mass, 5 to 80% by mass, 5 to 70% by mass, 5 to 60% by mass, 5 to 50% by mass, 5 to 40% by mass, 5 to 30% by mass; 10 to 97% by mass, 10 to 95% by mass, 10 to 90% by mass, 10 to 80% by mass, 10 to 70% by mass, 10 to 60% by mass, 10 to 50% by mass, 10 to 40% by mass, 10 to 30% by mass; 20 to 97% by mass, 20 to 95% by mass, 20 to 90% by mass, 20 to 80% by mass, 20 to 70% by mass, 20 to 60% by mass, 20 to 50% by mass, 20 to 40% by mass, and 20 to 30% by mass.

In the remover, the content of the metal compound relative to 1 part by mass of the polar base may be 0.0005 parts by mass or more, 0.001 parts by mass or more, 0.005 parts by mass or more, 0.01 parts by mass or more, 0.05 parts by mass or more, or 0.1 parts by mass or more. Within the range, denture adhesives are effectively removed.

In the remover, the content of the metal compound relative to 1 part by mass of the polar base may be 20 parts by mass or less, 10 parts by mass or less, 8 parts by mass or less, 5 parts by mass or less, 3 parts by mass or less, 2 parts by mass or less, 1 part by mass or less, or 0.5 parts by mass or less. Within the range, a sufficient amount of the polar base or other ingredients may be further contained, and the resulting formulation exhibits high efficacy in removing denture adhesives.

Examples of the content ratio of the metal compound relative to 1 part by mass of the polar base include 0.0005 to 20 parts by mass, 0.0005 to 10 parts by mass, 0.0005 to 8 parts by mass, 0.0005 to 5 parts by mass, 0.0005 to 3 parts by mass, 0.0005 to 2 parts by mass, 0.0005 to 1 part by mass, 0.0005 to 0.5 parts by mass; 0.001 to 20 parts by mass, 0.001 to 10 parts by mass, 0.001 to 8 parts by mass, 0.001 to 5 parts by mass, 0.001 to 3 parts by mass, 0.001 to 2 parts by mass, 0.001 to 1 part by mass, 0.001 to 0.5 parts by mass; 0.005 to 20 parts by mass, 0.005 to 10 parts by mass, 0.005 to 8 parts by mass, 0.005 to 5 parts by mass, 0.005 to 3 parts by mass, 0.005 to 2 parts by mass, 0.005 to 1 part by mass, 0.005 to 0.5 parts by mass; 0.01 to 20 parts by mass, 0.01 to 10 parts by mass, 0.01 to 8 parts by mass, 0.01 to 5 parts by mass, 0.01 to 3 parts by mass, 0.01 to 2 parts by mass, 0.01 to 1 part by mass, 0.01 to 0.5 parts by mass; 0.05 to 20 parts by mass, 0.05 to 10 parts by mass, 0.05 to 8 parts by mass, 0.05 to 5 parts by mass, 0.05 to 3 parts by mass, 0.05 to 2 parts by mass, 0.05 to 1 part by mass, 0.05 to 0.5 parts by mass; 0.1 to 20 parts by mass, 0.1 to 10 parts by mass, 0.1 to 8 parts by mass, 0.1 to 5 parts by mass, 0.1 to 3 parts by mass, 0.1 to 2 parts by mass, 0.1 to 1 part by mass, and 0.1 to 0.5 parts by mass.

When the metal compound is a hydrate, the concentration and the content of the metal compound are the concentration and the content of the compound excluding hydration water.

Polar bases and metal compounds formulated not for releasing denture adhesives, for example, used as the excipients described later also fall into the "polar base" and the "metal compound", respectively, in the present invention.

Although water is necessary for the remover of the present invention to exert the efficacy on the basis of the mechanism, the denture adhesive remover of the present invention may or may not comprise water. For a remover comprising water, preparation is not needed before use, and the remover may be directly applied to the oral cavity or a denture. Hence, the remover is highly convenient for use. For a remover comprising no water, preparation involving the addition of water is needed before use, but the absence of water reduces the weight of the formulation itself. Hence, the remover is highly convenient for distribution.

When water is contained, the concentration of the water relative to the total amount of the remover may be 0.001% by mass or more, 0.01% by mass or more, 0.1% by mass or more, 1% by mass or more, 5% by mass or more, 10% by mass or more, 20% by mass or more, 30% by mass or more, or 40% by mass or more. Within the range, the resulting formulation sufficiently exhibits the efficacy in removing denture adhesives without addition of water from outside.

The concentration of the water relative to the total amount of the remover may be 90% by mass or less, 80% by mass or less, 70% by mass or less, 60% by mass or less, or 50% by mass or less. Within the range, a sufficient amount of the metal compound or the polar base may be contained, and the resulting formulation sufficiently exhibits the efficacy in removing denture adhesives.

Examples of the concentration of the water relative to the total amount of the remover include 0.001 to 90% by mass, 0.001 to 80% by mass, 0.001 to 70% by mass, 0.001 to 60% by mass, 0.001 to 50% by mass; 0.01 to 90% by mass, 0.01 to 80% by mass, 0.01 to 70% by mass, 0.01 to 60% by mass, 0.01 to 50% by mass; 0.1 to 90% by mass, 0.1 to 80% by mass, 0.1 to 70% by mass, 0.1 to 60% by mass, 0.1 to 50% by mass; 1 to 90% by mass, 1 to 80% by mass, 1 to 70% by mass, 1 to 60% by mass, 1 to 50% by mass; 5 to 90% by mass, 5 to 80% by mass, 5 to 70% by mass, 5 to 60% by mass, 5 to 50% by mass; 10 to 90% by mass, 10 to 80% by mass, 10 to 70% by mass, 10 to 60% by mass, 10 to 50% by mass; 20 to 90% by mass, 20 to 80% by mass, 20 to 70% by mass, 20 to 60% by mass, 20 to 50% by mass; 30 to 90% by mass, 30 to 80% by mass, 30 to 70% by mass, 30 to 60% by mass, 30 to 50% by mass; 40 to 90% by mass, 40 to 80% by mass, 40 to 70% by mass, 40 to 60% by mass, and 40 to 50% by mass.

When the denture adhesive remover of the present invention comprises water, the content of the water relative to 1 part by mass of the polar base may be 0.001 parts by mass or more, 0.01 parts by mass or more, 0.05 parts by mass or more, 0.1 parts by mass or more, 1 part by mass or more, 5 parts by mass or more, 10 parts by mass or more, 30 parts by mass or more, 50 parts by mass or more, or 80 parts by mass or more. Within the range, irritation to the oral mucosa caused by the polar base is suppressed, and the resulting formulation sufficiently exhibits the efficacy in removing denture adhesives.

The content of the water relative to 1 part by mass of the polar base may be 95 parts by mass or less, 90 parts by mass or less, 80 parts by mass or less, 70 parts by mass or less, 60 parts by mass or less, 50 parts by mass or less, 30 parts by mass or less, 10 parts by mass or less, 5 parts by mass or less, or 3 parts by mass or less. Within the range, a sufficient amount of the metal compound or the polar base may be contained, and the resulting formulation sufficiently exhibits the efficacy in removing denture adhesives.

Examples of the content ratio of the water relative to 1 part by mass of the polar base include 0.001 to 95 parts by mass, 0.001 to 90 parts by mass, 0.001 to 80 parts by mass, 0.001 to 70 parts by mass, 0.001 to 60 parts by mass, 0.001 to 50 parts by mass, 0.001 to 30 parts by mass, 0.001 to 10 parts by mass, 0.001 to 5 parts by mass, 0.001 to 3 parts by mass; 0.01 to 95 parts by mass, 0.01 to 90 parts by mass, 0.01 to 80 parts by mass, 0.01 to 70 parts by mass, 0.01 to 60 parts by mass, 0.01 to 50 parts by mass, 0.01 to 30 parts by mass, 0.01 to 10 parts by mass, 0.01 to 5 parts by mass, 0.01 to 3 parts by mass; 0.05 to 95 parts by mass, 0.05 to 90 parts by mass, 0.05 to 80 parts by mass, 0.05 to 70 parts by mass, 0.05 to 60 parts by mass, 0.05 to 50 parts by mass, 0.05 to 30 parts by mass, 0.05 to 10 parts by mass, 0.05 to 5 parts by mass, 0.05 to 3 parts by mass; 0.1 to 95 parts by mass, 0.1 to 90 parts by mass, 0.1 to 80 parts by mass, 0.1 to 70 parts by mass, 0.1 to 60 parts by mass, 0.1 to 50 parts by mass, 0.1 to 30 parts by mass, 0.1 to 10 parts by mass, 0.1 to 5 parts by mass, 0.1 to 3 parts by mass; 1 to 95 parts by mass, 1 to 90 parts by mass, 1 to 80 parts by mass, 1 to 70 parts by mass, 1 to 60 parts by mass, 1 to 50 parts by mass, 1 to 30 parts by mass, 1 to 10 parts by mass, 1 to 5 parts by mass, 1 to 3 parts by mass; 5 to 95 parts by mass, 5 to 90 parts by mass, 5 to 80 parts by mass, 5 to 70 parts by mass, 5 to 60 parts by mass, 5 to 50 parts by mass, 5 to 30 parts by mass, 5 to 10 parts by mass; 10 to 95 parts by mass, 10 to 90 parts by mass, 10 to 80 parts by mass, 10 to 70 parts by mass, 10 to 60 parts by mass, 10 to 50 parts by mass, 10 to 30 parts by mass; 30 to 95 parts by mass, 30 to 90 parts by mass, 30 to 80 parts by mass, 30 to 70 parts by mass, 30 to 60 parts by mass, 30 to 50 parts by mass; 50 to 95 parts by mass, 50 to 90 parts by mass, 50 to 80 parts by mass, 50 to 70 parts by mass, 50 to 60 parts by mass; 80 to 95 parts by mass, and 80 to 90 parts by mass.

When the remover of the present invention comprises no water, water is added at the above concentration or content for preparation before use.

### Optional ingredients

The denture adhesive remover of the present invention may comprise excipients in addition to the polar base, the metal compound, and optional water, depending on the formulation type, to the extent not impairing the effect of the invention. Examples of the excipient include surfactants, pH adjusters, antibacterial ingredients or antiseptics, wetting agents, stabilizing agents, thickeners, sweetening agents, and flavoring agents. One or more excipients may be used.

As the surfactant, for example, an anionic surfactant, a cationic surfactant, an amphoteric surfactant, a nonionic surfactant, a naturally derived surfactant, and an organic solvent functioning as the solubilizer may be used.

The cleansing effect of the surfactant emulsifies a hydrophobic ointment base ingredient contained in denture adhesives, such as liquid paraffin and white petrolatum, and this further improves the efficiency of removing denture adhesives.

Examples of the anionic surfactant include higher alkyl sulfate salts such as sodium lauryl sulfate and potassium lauryl sulfate; alkyl ether sulfate salts such as triethanolamine polyoxyethylene(POE) lauryl sulfate and sodium polyoxyethylene(POE) lauryl sulfate; higher fatty acid amide sulfonate salts such as sodium methyl stearoyl taurate, sodium methyl myristoyl taurate, sodium methyl cocoyl taurate, and sodium taurine cocoyl methyltaurate; polyoxyethylene(POE) alkyl ether phosphates and salts thereof, such as sodium polyoxyethylene(POE) oleyl ether phosphate, sodium polyoxyethylene(POE) cetyl ether phosphate, and polyoxyethylene(POE) stearyl ether phosphate; phosphate esters and salts thereof, such as potassium cetyl phosphate and cetyl phosphate; sulfosuccinate salts such as sodium bis(2-ethylhexyl) sulfosuccinate; alkylbenzene sulfonate salts such as sodium linear-dodecylbenzenesulfonate; higher fatty acid ester sulfate salts such as sodium hydrogenated glyceryl cocoate sulfate; and N-acyl glutamate salts such as sodium lauroyl glutamate, sodium stearoyl glutamate, and sodium myristoyl glutamate.

Examples of the cationic surfactant include fatty acid amidoamines such as stearamidopropyl dimethylamine and behenamidopropyl dimethylamine; monoalkyl quaternary ammonium salts such as cetrimonium chloride, steartrimonium chloride, behentrimonium chloride, steartrimonium bromide, cetrimonium methosulfate, and behentrimonium methosulfate; monoalkyl ether quaternary ammonium salts such as stearoxypropyltrimonium chloride and behenyl PG-trimonium chloride; dialkyl quaternary ammonium salts such as distearyldimonium chloride, dicocodimonium chloride, dialkyl(C12-18) dimonium chloride, and quaternium-18; dialkyl ester quaternary ammonium salts such as dicocoylethyl hydroxyethylmonium methosulfate and distearoylethyl hydroxyethylmonium methosulfate; and benzalkonium quaternary ammonium salts such as benzalkonium chloride and benzethonium chloride.

Examples of the amphoteric surfactant include carbobetaine, sulfobetaine, imidazolinium betaine, and amide betaine.

Examples of the nonionic surfactant include polyoxyethylene(POE) alkyl ethers such as polyoxyethylene(POE) 2-decyltetradecyl alcohol, polyoxyethylene(POE) oleyl ether, and polyoxyethylene(POE) cetyl ether; sorbitan fatty acid esters such as sorbitan monostearate, sorbitan sesquistearate, sorbitan tristearate, sorbitan monooleate, and sorbitan sesquioleate; polyoxyethylene(POE) sorbitan fatty acid esters such as polyoxyethylene(POE) (20) sorbitan monoisostearate, polyoxyethylene(POE) (20) sorbitan monooleate, polyoxyethylene(POE) (20) sorbitan monostearate, polyoxyethylene(20) sorbitan monococoate, and polyoxyethylene(POE) (80) sorbitan laurate; glycerol fatty acid esters such as glyceryl monostearate and glyceryl oleate; polyoxyethylene(POE) glycerol fatty acid esters such as polyoxyethylene(POE) glyceryl stearate and polyoxyethylene glyceryl trioleate; polyoxyethylene dihydrocholesterol ester; polyoxyethylene(POE) hydrogenated castor oils such as polyoxyethylene hydrogenated castor oil 40(HCO-40), polyoxyethylene hydrogenated castor oil 50(HCO-50), polyoxyethylene hydrogenated castor oil 60(HCO-60), and polyoxyethylene hydrogenated castor oil 80; polyoxyethylene(POE) castor oils such as polyoxyethylene(POE) (3) castor oil and polyoxyethylene(POE) (20) castor oil; polyoxyethylene(POE) hydrogenated castor oil fatty acid esters such as polyoxyethylene(POE) hydrogenated castor oil isostearate; polyoxyethylene(POE) alkyl aryl ethers; alkyl glyceryl ethers such as cumyl alcohol, batyl alcohol, and selachyl alcohol; polyoxyethylene(POE) glyceryl alkyl ethers such as polyoxyethylene(POE) monostearyl glyceryl ether; polyoxyethylene(POE) fatty acid esters such as polyoxyl stearate; sucrose fatty acid esters such as sucrose palmitate, sucrose stearate, sucrose oleate, sucrose isostearate, sucrose linoleate, and sucrose linolenate; polyethylene glycol fatty acid esters such as polyethylene glycol dioleate; and polyglycerol fatty acid esters such as alkyl polyglycoside, diglyceryl monostearate, diglyceryl monoisostearate, decaglyceryl monostearate, decaglyceryl monooleate, decaglyceryl pentaoleate, and hexaglyceryl polyricinoleate.

Examples of the naturally derived surfactant include lecithin, hydrogenated lecithin, saponin, sodium surfactin, and bile acid.

Examples of the organic solvent include POE·POP alkyl ethers (the alkyl group has a carbon number of 10 to 26) (including POE·POP cetyl ethers such as POE(20) POP(4) cetyl ether, POE·POP decyl tetradecyl ethers such as POE(30) POP(6) decyl tetradecyl ether, and POE·POP decyl tetradecyl ethers such as POE(20) POP(6) decyl tetradecyl ether); POE alkyl ethers (the alkyl group has a carbon number of 10 to 26) (POE oleyl ethers such as POE(50) oleyl ether, POE oleyl ethers such as POE(20) oleyl ether, and POE lauryl ethers such as POE(25) lauryl ether); and solubilizers such as lauromacrogol.

When the remover of the present invention comprises a surfactant, the concentration of the surfactant relative to the total amount of the remover may be 0.01% by mass or more, 0.1% by mass or more, 1% by mass or more, 3% by mass or more, 5% by mass or more, or 10% by mass or more. Within the range, denture adhesives are more effectively removed.

When the remover of the present invention comprises a surfactant, the concentration of the surfactant relative to the total amount of the remover may be 20% by mass or less, 10% by mass or less, 8% by mass or less, 6% by mass or less, or 4% by mass or less. Typically, an excessive amount of a surfactant actually reduces the cleansing efficacy of a formulation. Within the range, however, the cleansing efficacy of the formulation is not reduced, and a sufficient amount of the metal compound or the polar base may be contained. Hence, the resulting formulation sufficiently exhibits the efficacy in removing denture adhesives.

Examples of the concentration of the surfactant relative to the total amount of the remover include 0.01 to 20% by mass, 0.01 to 10% by mass, 0.01 to 8% by mass, 0.01 to 6% by mass, 0.01 to 4% by mass; 0.1 to 20% by mass, 0.1 to 10% by mass, 0.1 to 8% by mass, 0.1 to 6% by mass, 0.1 to 4% by mass; 1 to 20% by mass, 1 to 10% by mass, 1 to 8% by mass, 1 to 6% by mass, 1 to 4% by mass; 3 to 20% by mass, 3 to 10% by mass, 3 to 8% by mass, 3 to 6% by mass, 3 to 4% by mass; 5 to 20% by mass, 5 to 10% by mass, 5 to 8% by mass, 5 to 6% by mass; and 10 to 20% by mass.

When the remover of the present invention comprises a surfactant, the content of the surfactant relative to 1 part by mass of the polar base may be 0.001 parts by mass or more, 0.005 parts by mass or more, 0.01 parts by mass or more, 0.05 parts by mass or more, 0.1 parts by mass or more, 1 part by mass or more, 5 parts by mass or more, or 10 parts by mass or more. Within the range, denture adhesives are effectively removed.

The content of the surfactant relative to 1 part by mass of the polar base may be 40 parts by mass or less, 30 parts by mass or less, 25 parts by mass or less, 20 parts by mass or less, 10 parts by mass or less, 5 parts by mass or less, 1 part by mass or less, or 0.5 parts by mass or less. Typically, an excessive amount of a surfactant actually reduces the cleansing efficacy of a formulation. Within the range, however, the cleansing efficacy of the formulation is not reduced, and a sufficient amount of the metal compound or the polar base may be contained. Hence, the resulting formulation sufficiently exhibits the efficacy in removing denture adhesives.

Examples of the content ratio of the surfactant relative to 1 part by mass of the polar base include 0.001 to 40 parts by mass, 0.001 to 30 parts by mass, 0.001 to 25 parts by mass, 0.001 to 20 parts by mass, 0.001 to 10 parts by mass, 0.001 to 5 parts by mass, 0.001 to 1 part by mass, 0.001 to 0.5 parts by mass; 0.005 to 40 parts by mass, 0.005 to 30 parts by mass, 0.005 to 25 parts by mass, 0.005 to 20 parts by mass, 0.005 to 10 parts by mass, 0.005 to 5 parts by mass, 0.005 to 1 part by mass, 0.005 to 0.5 parts by mass; 0.01 to 40 parts by mass, 0.01 to 30 parts by mass, 0.01 to 25 parts by mass, 0.01 to 20 parts by mass, 0.01 to 10 parts by mass, 0.01 to 5 parts by mass, 0.01 to 1 part by mass, 0.01 to 0.5 parts by mass; 0.05 to 40 parts by mass, 0.05 to 30 parts by mass, 0.05 to 25 parts by mass, 0.05 to 20 parts by mass, 0.05 to 10 parts by mass, 0.05 to 5 parts by mass, 0.05 to 1 part by mass, 0.05 to 0.5 parts by mass; 0.1 to 40 parts by mass, 0.1 to 30 parts by mass, 0.1 to 25 parts by mass, 0.1 to 20 parts by mass, 0.1 to 10 parts by mass, 0.1 to 5 parts by mass, 0.1 to 1 part by mass, 0.1 to 0.5 parts by mass; 1 to 40 parts by mass, 1 to 30 parts by mass, 1 to 25 parts by mass, 1 to 20 parts by mass, 1 to 10 parts by mass, 1 to 5 parts by mass; 5 to 40 parts by mass, 5 to 30 parts by mass, 5 to 25 parts by mass, 5 to 20 parts by mass, 5 to 10 parts by mass, 1 to 5 parts by mass; 10 to 40 parts by mass, 10 to 30 parts by mass, 10 to 25 parts by mass, and 10 to 20 parts by mass.

When the remover of the present invention comprises a surfactant, the content of the surfactant relative to 1 part by mass of the metal compound may be 0.01 parts by mass or more, 0.05 parts by mass or more, 0.1 parts by mass or more, 0.15 parts by mass or more, 0.5 parts by mass or more, 0.8 parts by mass or more, or 1 part by mass or more. Within the range, denture adhesives are effectively removed.

The content of the surfactant relative to 1 part by mass of the metal compound may be 20 parts by mass or less, 10 parts by mass or less, 5 parts by mass or less, or 3 parts by mass or less. Typically, an excessive amount of a surfactant actually reduces the cleansing efficacy of a formulation. Within the range, however, the cleansing efficacy of the formulation is not reduced, and a sufficient amount of the metal compound or the polar base may be contained. Hence, the resulting formulation sufficiently exhibits the efficacy in removing denture adhesives.

Examples of the content ratio of the surfactant relative to 1 part by mass of the metal compound include 0.01 to 20 parts by mass, 0.01 to 10 parts by mass, 0.01 to 5 parts by mass, 0.01 to 3 parts by mass; 0.05 to 20 parts by mass, 0.05 to 10 parts by mass, 0.05 to 5 parts by mass, 0.05 to 3 parts by mass; 0.1 to 20 parts by mass, 0.1 to 10 parts by mass, 0.1 to 5 parts by mass, 0.1 to 3 parts by mass; 0.15 to 20 parts by mass, 0.15 to 10 parts by mass, 0.15 to 5 parts by mass, 0.15 to 3 parts by mass; 0.5 to 20 parts by mass, 0.5 to 10 parts by mass, 0.5 to 5 parts by mass, 0.5 to 3 parts by mass; 0.8 to 20 parts by mass, 0.8 to 10 parts by mass, 0.8 to 5 parts by mass, 0.8 to 3 parts by mass; 1 to 20 parts by mass, 1 to 10 parts by mass, 1 to 5 parts by mass, and 1 to 3 parts by mass.

Examples of the pH adjuster include organic acids such as acetic acid, oxalic acid, maleic acid, fumaric acid, malonic acid, lactic acid, tartaric acid, citric acid, benzoic acid, methanesulfonic acid, succinic acid, malic acid, ascorbic acid, adipic acid, and benzoic acid, and salts thereof; inorganic acids such as phosphoric acid, hydrochloric acid, nitric acid, and carbonic acid, and salts thereof; inorganic bases such as potassium hydroxide and sodium hydroxide; and organic bases such as triethanolamine, diisopropanolamine, and triisopropanolamine.

Examples of the antibacterial ingredient or antiseptic include glycerol fatty acid esters; parabens such as isobutyl p-hydroxybenzoate, isopropyl p-hydroxybenzoate, methyl p-hydroxybenzoate, sodium methyl p-hydroxybenzoate, ethyl p-hydroxybenzoate, sodium ethyl p-hydroxybenzoate , butyl p-hydroxybenzoate, propyl p-hydroxybenzoate, and sodium propyl p-hydroxybenzoate; benzoic acids such as benzoic acid, sodium benzoate, and benzyl benzoate; quaternary ammonium salts such as benzalkonium chloride, benzethonium chloride, methylbenzethonium chloride, cetylpyridinium chloride, tetraethylammonium bromide, and didecyldimethylammonium chloride; ethylenediaminetetraacetate; chlorhexidine salts such as chlorhexidine gluconate and chlorhexidine hydrochloride; isopropylmethylphenol; and hinokitiol.

Examples of the wetting agent include glycerol and sorbitol.

Examples of the stabilizing agent include sodium polyacrylate, dibutylhydroxytoluene, and butylated hydroxyanisole.

Examples of the thickener include guar gum, locust bean gum, carrageenan, xanthan gum, polyvinyl alcohol, polyvinylpyrrolidone, carboxyvinyl polymer, acrylic acid-alkyl methacrylate copolymers, polyethylene glycol, bentonite, alginic acid, macrogol, and cellulose thickeners (such as methyl cellulose, ethyl cellulose, hydroxyethyl cellulose, hydroxymethyl cellulose, hydroxypropyl cellulose, hydroxypropyl methyl cellulose, carboxymethyl cellulose, and carboxyethyl cellulose).

Examples of the sweetening agent include saccharin sodium, stevia extract, sucralose, palatinose, maltitol, xylitol, trehalose, and arabitol.

Examples of the flavoring agent include various essential oils, essential oil ingredients such as menthol, and fragrance blends.

The remover of the present invention may be in the state of solid such as a powder, tablets, and gum, liquid (including a viscous fluid), or paste. The remover may be a sheet formulation in which a liquid or a paste-like formulation is infiltrated in a nonwoven fabric. When the remover is for the oral cavity described later, a gum-like, a paste-like, or a sheet-like formulation is preferred to prevent elderly people from accidentally swallowing the formulation.

When the remover is a dry solid formulation such as a powder or tablets, the remover may be an extemporaneous formulation that is mixed with water or the like before use. When the remover is an extemporaneous formulation, the composition of the remover of the present invention described above is the composition of the final preparation as a mixture with water or the like for use.

The remover of the present invention may be a denture adhesive remover for removing a denture adhesive from a denture. The remover may also be a denture adhesive remover for removing a denture adhesive from the oral cavity (the mucosa in the oral cavity), or a denture adhesive remover for the oral cavity. The denture adhesive remover is sometimes referred to as a denture adhesive cleanser.

The dosage of the remover of the present invention may be 1.5 to 15 g, specifically 2 to 10 g, relative to 1 g of a denture adhesive. The remover of the present invention is applied (for example, by coating, spraying, or pasting) to a denture adhesive, and then, for example, the denture adhesive is moved by hand, or the denture adhesive is rubbed by a finger, a brush, a cloth, or the like. This removes the denture adhesive from a denture or the oral cavity. When the remover of the present invention is applied to a denture adhesive and then is allowed to stand for 60 seconds or less, specifically for 5 to 10 seconds, the tackiness of the denture adhesive is more effectively reduced, and this facilitates the removement.

The present invention encompasses a method for removing a denture adhesive (for example, a method for removing a denture adhesive from a denture or the oral cavity). The method comprises a step of applying a composition comprising a polar base and at least one metal compound, to a denture adhesive. In the method, the metal compound is a compound selected from the group consisting of:
(1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
(2) an oxide of at least one metal selected from the group consisting of sodium, potassium, calcium, magnesium, aluminum, zinc, and iron, or a hydrate thereof; and
(3) a hydroxide of at least one metal selected from the group consisting of sodium, potassium, calcium, magnesium, aluminum, zinc, and iron, or a hydrate thereof.

In the metal compound, two or more metals may be contained, and two or more counter ions may be contained for a metal ion. Examples of the metal compound include salts of the metal with an organic acid or an inorganic acid and with ammonia.

In the method, the denture adhesive may adhere to a denture or the oral cavity. The composition, the state, and the like of the composition comprising a polar base and a metal compound are as described for the denture adhesive remover of the present invention.

The dosage of the composition comprising a polar base and at least one metal compound may be an effective dose and may be 1.5 to 15 g, specifically 2 to 10 g, relative to 1 g of a denture adhesive. Examples of the method of applying the remover of the present invention to a denture adhesive include coating, spraying, and pasting. After the applying step, the method may comprise a step of allowing the denture adhesive with the composition to stand, and the denture adhesive with the composition may be allowed to stand for 60 seconds or less, specifically for 5 to 10 seconds. The method may further comprise a step of removing the denture adhesive from a denture or the oral cavity by applying force to the denture adhesive.

### EXAMPLES

The present invention will next be described in further detail with reference to Examples, but the present invention is not limited to them.

### (1) Preparation of denture adhesive remover

Liquid or powdery formulations having the compositions shown in Table 1 to 7 were prepared by mixing ingredients.

**[Table 1]**

| Type of metal compound | | | | | | | | | | | | | | | | | |
|---|---|---|---|---|---|---|---|---|---|---|---|---|---|---|---|---|---|
| (unit: % by mass) | | | | | | | | | | | | | | | | | |
| | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 | Example 17 |
| Calcium chloride | 1 | 29 | 5 | 5 | - | - | - | - | - | - | - | - | - | - | - | - | - |
| Calcium lactate | - | - | - | - | 5 | - | - | - | - | - | - | - | - | - | - | - | - |
| Sodium chloride | - | - | - | - | - | 5 | - | - | - | - | - | - | - | - | - | - | - |
| Magnesium chloride hexahydrate | - | - | - | - | - | - | 5 | - | - | - | - | - | - | - | - | - | - |
| Zinc chloride | - | - | - | - | - | - | - | 5 | - | - | - | - | - | - | - | - | - |
| Iron chloride (anhydrous) | - | - | - | - | - | - | - | - | 5 | - | - | - | - | - | - | - | - |
| Sodium carbonate | - | - | - | - | - | - | - | - | - | 5 | - | - | - | - | - | - | - |
| Potassium fluoride | - | - | - | - | - | - | - | - | - | - | 5 | - | - | - | - | - | - |
| Zinc fluoride | - | - | - | - | - | - | - | - | - | - | - | 5 | - | - | - | - | - |
| Aluminum phosphate | - | - | - | - | - | - | - | - | - | - | - | - | 5 | - | - | - | - |
| Sodium thiosulfate | - | - | - | - | - | - | - | - | - | - | - | - | - | 5 | - | - | - |
| Anhydrous sodium sulfate | - | - | - | - | - | - | - | - | - | - | - | - | - | - | 5 | - | - |
| Magnesium sulfate | - | - | - | - | - | - | - | - | - | - | - | - | - | - | - | 5 | - |
| Aluminum sulfate | - | - | - | - | - | - | - | - | - | - | - | - | - | - | - | - | 5 |
| Purified water | 69 | 41 | 15 | - | 65 | 65 | 65 | 65 | 65 | 65 | 65 | 65 | 65 | 65 | 65 | 65 | 65 |
| Macrogol 400 | 30 | - | 15 | 15 | 30 | 30 | 30 | 30 | 30 | 30 | 30 | 30 | 30 | 30 | 30 | 30 | 30 |
| Macrogol 4000 | - | - | 65 | 80 | - | - | - | - | - | - | - | - | - | - | - | - | - |
| Ethanol | - | 30 | - | - | - | - | - | - | - | - | - | - | - | - | - | - | - |
| State | liquid | liquid | paste | solid | liquid | liquid | liquid | liquid | liquid | liquid | liquid | liquid | liquid | liquid | liquid | liquid | liquid |
| Removability of denture adhesive from denture | 4 | 4 | 4 | 3 | 3 | 4 | 4 | 4 | 4 | 2 | 2 | 4 | 2 | 2 | 2 | 2 | 4 |
| Irritation to oral mucosa/ unpleasant taste * | ○ | × | Δ | Δ | ○ | ○ | × | × | Δ | Δ | × | × | × | × | Δ | Δ | × |
| Removability of denture adhesive from oral cavity | suitable | - | suitable | suitable | suitable | suitable | - | - | suitable | suitable | - | - | - | - | suitable | suitable | - |

| | | | | | | | | | | | | | | | | | |
|---|---|---|---|---|---|---|---|---|---|---|---|---|---|---|---|---|---|
| * A remover with neither irritation nor unpleasant taste is evaluated as ∘; a remover that has irritation or unpleasant taste but is tolerable for use in the oral cavity is evaluated as △; and a remover that has irritation or unpleasant taste and is difficult to use in the oral cavity is evaluated as × 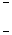 . | | | | | | | | | | | | | | | | | |

**[Table 2]**

| Type of metal compound | | | | | | | | | | | | | | | | | |
|---|---|---|---|---|---|---|---|---|---|---|---|---|---|---|---|---|---|
| (unit: % by mass) | | | | | | | | | | | | | | | | | |
| | Example 18 | Example 19 | Example 20 | Example 21 | Example 22 | Example 23 | Example 24 | Example 25 | Example 26 | Example 27 | Example 28 | Example 29 | Example 30 | Example 31 | Example 32 | Example 33 | Example 34 |
| Iron sulfate heptahydrate | 5 | - | - | - | - | - | - | - | - | - | - | - | - | - | - | - | - |
| Sodium sulfite | - | 5 | - | - | - | - | - | - | - | - | - | - | - | - | - | - | - |
| Calcium nitrate tetrahydrate | - | - | 5 | - | - | - | - | - | - | - | - | - | - | - | - | - | - |
| Magnesium nitrate | - | - | - | 5 | - | - | - | - | - | - | - | - | - | - | - | - | - |
| Zinc nitrate hexahydrate | - | - | - | - | 5 | - | - | - | - | - | - | - | - | - | - | - | - |
| Aluminum lactate | - | - | - | - | - | 5 | - | - | - | - | - | - | - | - | - | - | - |
| Potassium acetate | - | - | - | - | - | - | 5 | - | - | - | - | - | - | - | - | - | - |
| Calcium acetate monohydrate | - | - | - | - | - | - | - | 5 | - | - | - | - | - | - | - | - | - |
| Magnesium acetate tetrahydrate | - | - | - | - | - | - | - | - | 5 | - | - | - | - | - | - | - | - |
| Potassium hexacyanoferrate | - | - | - | - | - | - | - | - | - | 5 | - | - | - | - | - | - | - |
| Sodium hydroxide | - | - | - | - | - | - | - | - | - | - | 5 | - | - | - | - | - | - |
| Potassium hydroxide | - | - | - | - | - | - | - | - | - | - | - | 5 | - | - | - | - | - |
| Calcium oxide | - | - | - | - | - | - | - | - | - | - | - | - | 5 | - | - | - | - |
| Aluminum oxide | - | - | - | - | - | - | - | - | - | - | - | - | - | 5 | - | - | - |
| Aluminum ammonium sulfate | - | - | - | - | - | - | - | - | - | - | - | - | - | - | 5 | - | - |
| Ammonium iron sulfate | - | - | - | - | - | - | - | - | - | - | - | - | - | - | - | 5 | - |
| Aluminum potassium sulfate | - | - | - | - | - | - | - | - | - | - | - | - | - | - | - | - | 5 |
| Purified water | 65 | 65 | 65 | 65 | 65 | 65 | 65 | 65 | 65 | 65 | 65 | 65 | 65 | 65 | 65 | 65 | 65 |
| Macrogol 400 | 30 | 30 | 30 | 30 | 30 | 30 | 30 | 30 | 30 | 30 | 30 | 30 | 30 | 30 | 30 | 30 | 30 |
| State | liquid | liquid | liquid | liquid | liquid | liquid | liquid | liquid | liquid | liquid | liquid | liquid | liquid | liquid | liquid | liquid | liquid |
| Removability of denture adhesive from denture | 4 | 2 | 4 | 4 | 4 | 3 | 2 | 4 | 4 | 4 | 3 | 3 | 3 | 2 | 4 | 4 | 4 |
| Irritation to oral mucosa/ unpleasant taste * | Δ | Δ | × | Δ | ○ | ○ | ○ | ○ | Δ | × | × | × | × | ○ | Δ | Δ | Δ |
| Removability of denture adhesive from oral cavity | suitable | suitable | - | suitable | suitable | suitable | suitable | suitable | suitable | - | - | - | - | suitable | suitable | suitable | suitable |

| | | | | | | | | | | | | | | | | | |
|---|---|---|---|---|---|---|---|---|---|---|---|---|---|---|---|---|---|
| * A remover with neither irritation nor unpleasant taste is evaluated as ∘; a remover that has irritation or unpleasant taste but is tolerable for use in the oral cavity is evaluated as △; and a remover that has irritation or unpleasant taste and is difficult to use in the oral cavity is evaluated as × 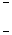 . | | | | | | | | | | | | | | | | | |

**[Table 3]**

| Concentration of metal compound | | | | | | | | | |
|---|---|---|---|---|---|---|---|---|---|
| (unit: % by mass) | | | | | | | | | |
| | Example 35 | Example 36 | Example 37 | Example 38 | Example 39 | Example 40 | Example 41 | Example 42 | Example 43 |
| Calcium chloride | 0.1 | 1 | 1 | 3 | 25 | 50 | 50 | 70 | 90 |
| Purified water | 49.9 | 49 | - | 47 | 37.5 | 20 | 35 | 15 | - |
| Macrogol 400 | 50 | 50 | 99 | 50 | 37.5 | 30 | 15 | 15 | 10 |
| State | liquid | liquid | liquid | liquid | liquid | paste | liquid | paste | solid |
| Removability of denture adhesive from denture | 3 | 4 | 2 | 4 | 3 | 2 | 2 | 2 | 2 |
| Irritation to oral mucosal unpleasant taste * | ○ | ○ | Δ | ○ | × | × | × | × | × |
| Removability of denture adhesive from oral cavity | suitable | suitable | suitable | suitable | - | - | - | - | - |

| | | | | | | | | | |
|---|---|---|---|---|---|---|---|---|---|
| * A remover with neither irritation nor unpleasant taste is evaluated as ∘; a remover that has irritation or unpleasant taste but is tolerable for use in the oral cavity is evaluated as △; and a remover that has irritation or unpleasant taste and is difficult to use in the oral cavity is evaluated as × 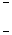 . | | | | | | | | | |

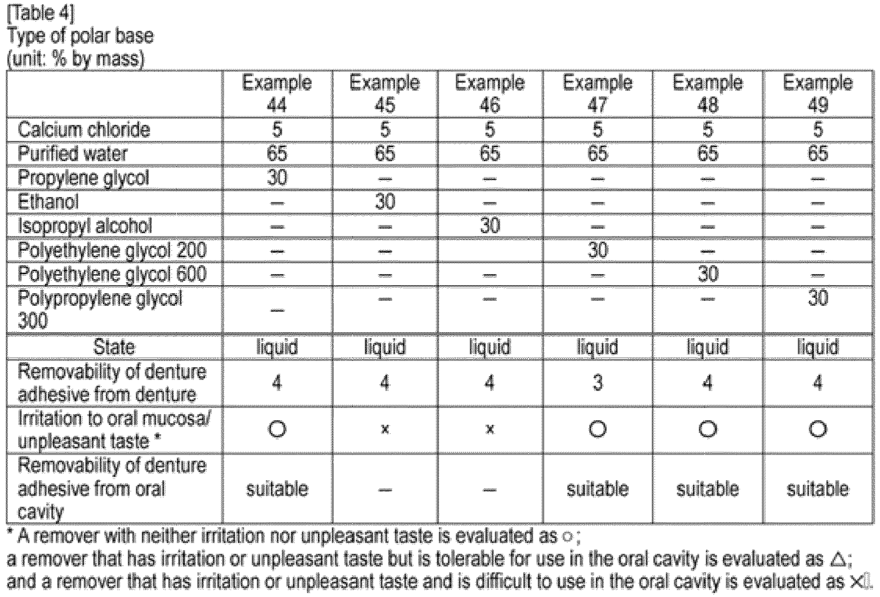

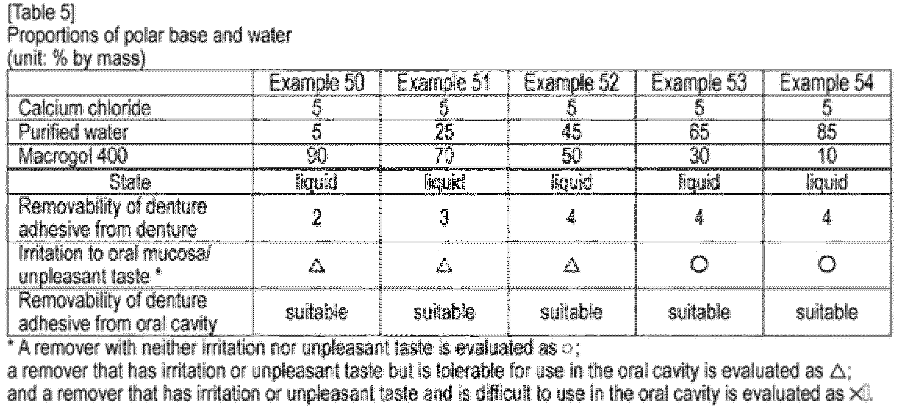

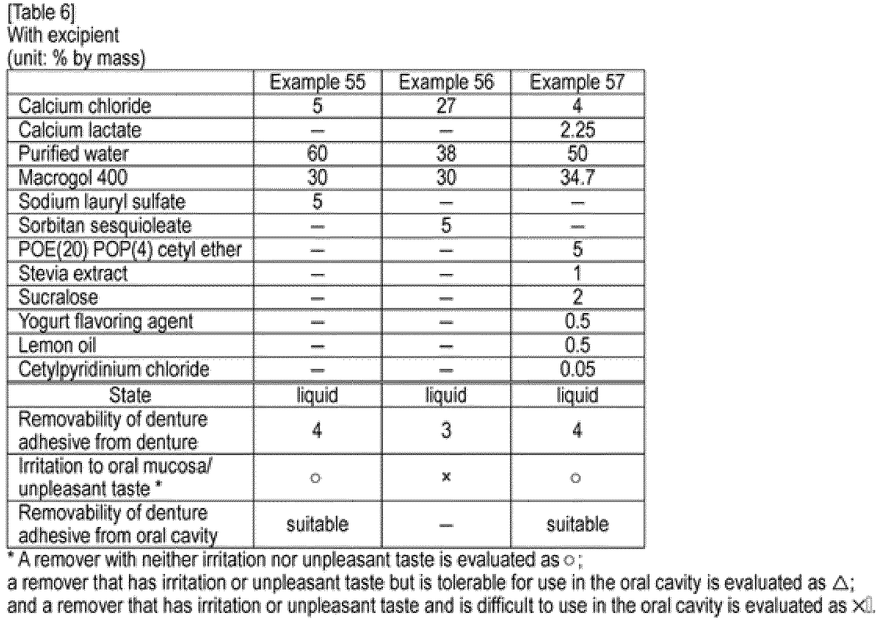

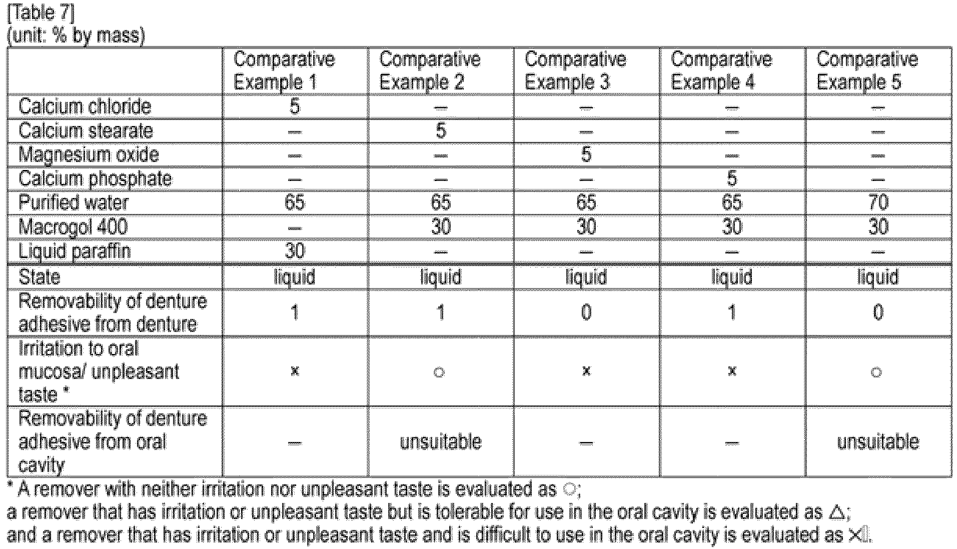

### (2) Performance evaluation of denture adhesive remover 1

By simulating the conditions of a denture and the oral cavity, the performance of a denture adhesive remover was evaluated. A glass plate measuring 70 × 70 mm and 2 mm thick was coated with 0.1 g of a denture adhesive (trade name: Super Poligrip Free), and the whole was immersed in tap water. After 1 minute, the plate was pulled up. Another glass plate having the same size was then placed on the denture adhesive, and a 1-kg weight was placed to apply a load. After 10 seconds, the weight was removed, and one of the glass plates was peeled off to prepare a sample. The denture adhesive as the sample and the periphery of the sample were coated with 1 g of the formulation in each of Examples and Comparative Examples. Immediately after the coating, the sample was rubbed by a finger, and the condition of the released denture adhesive was scored on a five-point scale on the basis of the following criteria. A remover having a score of 2 or more was evaluated as "acceptance" and was determined to be able to sufficiently remove the denture adhesive effectively and efficiency.
0: Not removed
1: Applying force moves the surface layer of the denture adhesive but fails to release the denture adhesive.
2: Applying a small force moves the surface layer of the denture adhesive, and applying a strong force enables almost complete release.
3: Applying a strong force enables complete release.
4: Applying a small force enables complete release.

Evaluation results are shown in Tables 1 to 7.

The formulation in Comparative Example 1 comprising no polar base, the formulations in Comparative Examples 2 to 4 comprising, as the metal compound, calcium stearate, magnesium oxide, and calcium phosphate, respectively, and the formulation in Comparative Example 5 comprising no metal compound failed to remove the denture adhesive. On the other hand, the formulations in Examples 1 to 57 successfully removed the denture adhesive.

The remover of the present invention works to change physical properties of a denture adhesive and accordingly facilitates the release of the denture adhesive. Hence, it is reasonably predicted that a remover capable of removing the denture adhesive from a glass plate enables removal from the oral cavity. Some of the formulations in Examples cause irritation or have unpleasant taste and thus are unsuitable for removing a denture adhesive from the oral cavity but are useful for removing a denture adhesive from a denture.

### (3) Performance evaluation of denture adhesive remover 2

The formulations in Examples and Comparative Examples determined to be suited for use in the oral cavity from the viewpoint of irritation, taste, and the like (Examples and Comparative Examples in which irritation to the oral mucosa/unpleasant taste is ∘ or △ in Tables 1 to 7) were evaluated for the performance in removing a denture adhesive in the human oral cavity. The palate of one subject was coated with 0.2 g of a denture adhesive (trade name: Super Poligrip Free) colored with Blue No. 1(Brilliant Blue FCF), and a splint prepared to fit the palate of the subject was attached to cover the denture adhesive. Ten minutes after the attachment, the splint was detached. The denture adhesive remaining on the palate was tried to be removed by cleansing the palate using a nonwoven fabric impregnated with 1 g of the formulation in each of Examples and Comparative Examples wrapped around a finger. The cleansing time was 30 seconds, and a remover capable of removing the denture adhesive was evaluated as "suitable". The formulation in Comparative Example 2 comprised ingredients neither causing irritation nor having unpleasant taste.

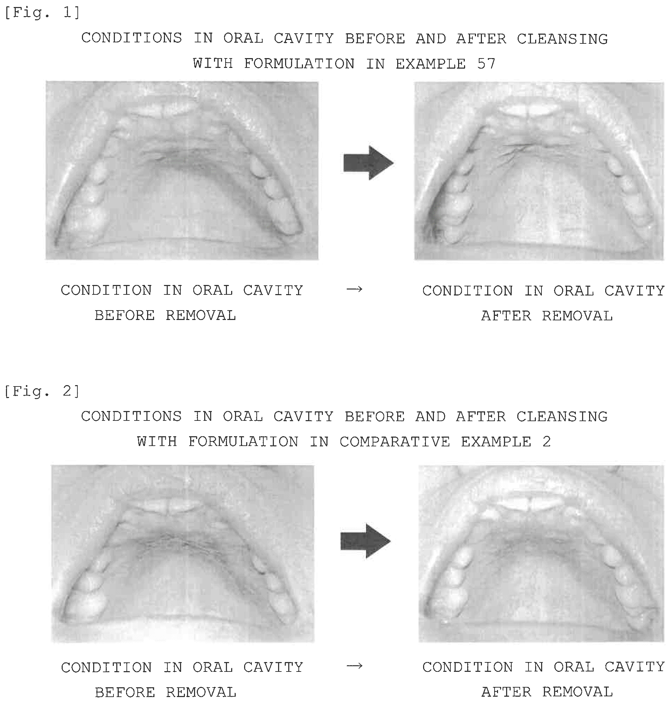

The results are shown in Table 1 to 7. The formulations in Examples in which irritation to the oral mucosa/unpleasant taste was o or △ were able to completely remove the denture adhesive adhering to the oral cavity without causing injury to the oral mucosa. Fig. 1 shows the conditions in the oral cavity before and after cleansing with the formulation in Example 57. Substantially the same conditions were observed with the formulations in other Examples. Fig. 2 shows the conditions in the oral cavity before and after cleansing with the formulation in Comparative Example 2. When the formulation in Comparative Example 2 was used, the tackiness of the denture adhesive was not reduced, and the denture adhesive was not sufficiently removed by cleansing for 30 seconds. A thin layer of the denture adhesive remained in the oral cavity.

### Industrial Applicability

The remover of the present invention comprises a polar base and a metal compound, which chemically reduce the tackiness of a denture adhesive, and thus can effectively and efficiently remove the denture adhesive from the oral cavity and a denture. The remover can be prepared by using general-purpose ingredients usable in the oral cavity due to less irritation and less unpleasant taste and thus is applicable in various fields such as dental care and nursing care regions, providing a high commercial value.

## Claims

1. A denture adhesive remover comprising a polar base and at least one metal compound, wherein the metal compound is a compound selected from the group consisting of:
(1) a salt of at least one metal selected from the group consisting of sodium, potassium, calcium, magnesium, aluminum, zinc, and iron with at least one compound selected from the group consisting of inorganic acids, organic acids, and ammonia, or a hydrate thereof;
(2) an oxide of at least one metal selected from the group consisting of sodium, potassium, calcium, magnesium, aluminum, zinc, and iron, or a hydrate thereof; and
(3) a hydroxide of at least one metal selected from the group consisting of sodium, potassium, calcium, magnesium, aluminum, zinc, and iron, or a hydrate thereof.

2. The denture adhesive remover according to claim 1, wherein the inorganic acid is a compound selected from the group consisting of hydrochloric acid, carbonic acid, nitric acid, nitrous acid, sulfuric acid, sulfurous acid, thiosulfuric acid, silicic acid, phosphoric acid, and hydrofluoric acid, and the organic acid is a compound selected from the group consisting of lactic acid, acetic acid, citric acid, gluconic acid, and hexacyanoferric acid.

3. The denture adhesive remover according to claim 1 or 2, wherein the polar base is at least one selected from the group consisting of lower alcohols, higher alcohols, polyhydric alcohols, and glycol ethers.

4. The denture adhesive remover according to any one of claims 1 to 3, wherein the concentration of the polar base is 1 to 99% by mass relative to the total amount of the denture adhesive remover.

5. The denture adhesive remover according to any one of claims 1 to 4, wherein the concentration of the metal compound is 0.01 to 97% by mass relative to the total amount of the denture adhesive remover.

6. The denture adhesive remover according to any one of claims 1 to 5, wherein the content of the metal compound is 0.0005 to 20 parts by mass relative to 1 part by mass of the polar base.

7. The denture adhesive remover according to any one of claims 1 to 6, further comprising a surfactant.

8. The denture adhesive remover according to claim 7, wherein the concentration of the surfactant is 0.01 to 20% by mass relative to the total amount of the denture adhesive remover.

9. The denture adhesive remover according to claim 7 or 8, wherein the content of the surfactant is 0.01 to 20 parts by mass relative to 1 part by mass of the metal compound.

10. The denture adhesive remover according to any one of claims 1 to 9, further comprising water.

11. The denture adhesive remover according to claim 10, wherein the concentration of the water is 0.001 to 90% by mass relative to the total amount of the denture adhesive remover.

12. The denture adhesive remover according to any one of claims 1 to 11, for removing a denture adhesive from a denture or the oral cavity.
